# EUROPEAN PATENT SPECIFICATION

(11) **EP 2 142 658 B1**
(45) Date of publication and mention of the grant of the patent: **07.09.2011**
(21) Application number: 08719055.9
(22) Date of filing: 21.03.2008
(51) Int. Cl.: C12P 19/14, C13K 1/06, B01F 5/04, C12M 1/40, B01F 3/12, B01F 7/16, B01F 13/10

(54) **Liquefaction of starch-based biomass**
Verflüssigung von stärkehaltiger Biomasse
Liquéfaction de biomasse à base d'amidon

(30) Priority: 02.05.2007 GB 0708482; 05.06.2007 GB 0710659
(43) Date of publication of application: 13.01.2010
(73) Proprietor: Pursuit Dynamics PLC., Hinchingbrooke Business Park Huntingdon Cambridgeshire PE29 6HB (GB)
(72) Inventor: THORUP, Jens, Havon, Suffolk IP32 7GB (GB); HEATHCOTE, John, Gervase, Mark, Huntingdon Cambridgeshire PE29 6HB (GB); FENTON, Marcus, Brian, Mayhall, Cambridgeshire PE19 8BP (GB)
(74) Representative: Hendry, Niall James
(86) International application number: PCT/GB2008/050210
(87) International publication number: WO 2008/135775

(56) References cited:
- GB-A- 1 028 211

## Description

The present invention is a biomass treatment process suitable for use in biofuel production. More specifically, the present invention relates to an improved process and apparatus for converting starch-based biomass into sugars for subsequent fermentation.

The process of converting starch-based biomass into sugars is known in biofuel production as liquefaction. This liquefaction process involves extracting starch from starch-based feedstock and then converting the extracted starch into short chain polysaccharides for subsequent saccharification, fermentation and distillation into alcohol such as ethanol, for example.

Liquefaction processes typically involve an initial hydration step of mixing ground starch-based feedstock with water to form a slurry. The water may be pre-heated prior to being mixed with the feedstock. The slurry may additionally be heated in a vessel in order to activate the starch, and is then heated again and mixed with a liquefaction enzyme in order to convert the starch to short chain sugars. The activation stage typically uses steam-jacketed tanks or steam sparge heating to heat the slurry to the desired temperature. At the same time agitation mixers, slurry recirculation loops, or a combination of the two mix the slurry. However, despite the presence of the recirculation pumps these heating methods can result in regions being created in the slurry tank or vessel whose temperature is much greater than the remainder of the tank. In such "slow release" processes, starch hydrated early in the process can be damaged if it comes into contact with these high temperature regions, resulting in a lower yield. These arrangements also do not provide particularly efficient mixing, as evidenced by the heat damage problem discussed above. These conventional processes normally use separate vessels for the activation and conversion stages of the process. Transfer of the slurry from the activation vessel to the conversion stage vessel is normally accomplished using centrifugal pumps, which impart a high shear force on the slurry and cause further damage to the hydrated starch as a result.

The conversion stage may also use steam- or water-jacketed tanks, or tanks heated by sparge heaters, to raise the temperature of the slurry to the appropriate level for the optimum performance of the enzyme. Alternatively, jet cookers are employed to heat the incoming slurry into the conversion stage vessel.. Not only can the slurry suffer the same heat damage as in the activation stage, but the high temperature regions also contribute to limiting the glucose yield from the process. The excessive heat of these regions promotes Maillard reactions, where the sugar molecules are destroyed due to interaction with proteins also present in the slurry. The combination of these Maillard losses with the shear losses from the transfer pumps limits the glucose yield available. Additionally, existing liquefaction processes require a long residence time for the slurry in the conversion stage to ensure that as much starch is converted to sugars as possible. This leads to a longer production process with increased costs

It is an aim of the present invention to mitigate or obviate one or more of these disadvantages.

Two patents disclose a process (GB1028211) and an according apparatus (GB995660) for making glucose or maltose from starch, wherein an aqueous suspension of starch is mixed with alpha-amylase in a tank, and subjected to gelatinization and liquefaction in a vessel, wherein it is heated to 95 °C by mixing with steam coming from a pipe and subjected to impacting and/or shearing forces. The apparatus comprises a tube having at least one constriction through which a starch suspension is passed, and having in the tube wall, beyond the constriction in the transition region to full cross-section, one or more inlet orifices for the steam or chemical solution.

According to a first aspect of the present invention, there is provided a process for the treatment of a starch-based feedstock, comprising:
mixing together starch-based feedstock and working fluid to form a slurry;
hydrating the starch-based feedstock with the working fluid;
adding a liquefaction enzyme to the slurry and pumping the slurry into a substantially constant diameter passage of a starch activation device; and
further hydrating the starch-based feedstock and activating the starch content of the slurry by injecting a high velocity transport fluid into the slurry through a nozzle communicating with the passage;
wherein the working fluid is water and the transport fluid is steam.

The injection of the transport fluid into the slurry may:
apply a shear force to the slurry;
atomise the liquid phase within the slurry to create a dispersed droplet flow regime;
form a low pressure region downstream of the nozzle; and
generate a condensation shock wave within the passage downstream of the nozzle by condensation of the transport fluid.

The first hydrating step may include heating the slurry within a first vessel and/or maintaining it at a first predetermined temperature for a first predetermined period of time.

The process may further comprise the step of transferring the slurry to a second vessel from the starch activation device, and maintaining the temperature of the slurry in the second vessel for a second predetermined period of time.

The step of transferring the slurry to the second vessel may include passing the slurry through a temperature conditioning unit to raise the temperature of the slurry.

The step of heating the slurry in the first and second vessels may also include the step of agitating the slurry for the respective first and second periods of time.

The activation step may include recirculating the slurry through the starch activation device.

The transport fluid may be injected at a supersonic velocity.

The step of injecting the transport fluid may comprise injecting the high velocity transport fluid into the slurry through a plurality of nozzles communicating with the passage. The step of injecting the transport fluid into the slurry may occur on a single pass of the slurry through the starch activation device.

The pumping of the slurry may be carried out using a low shear pump.

According to a second aspect of the present invention there is provided an apparatus for treating a starch-based feedstock, the apparatus comprising:
hydrating means for mixing and hydrating the feedstock with a working fluid to form a slurry; and
a starch activation device in fluid communication with the first hydrating means;
wherein the starch activation device comprises:
a passage of substantially constant diameter having an inlet in fluid communication with the first hydrating means and an outlet; and
a transport fluid nozzle communicating with the passage and adapted to inject high velocity transport fluid into the passage.

The hydrating means may comprise a first vessel having an outlet in fluid communication with the inlet of the passage. The hydrating means may comprise a heating means for heating the working fluid and/or the slurry.

The apparatus may further comprise a second vessel having an inlet in fluid communication with the outlet of the passage. The second vessel may include insulating means for insulating the contents of the second vessel.

Alternatively, the apparatus may further comprise a residence tube section having an inlet in fluid communication with the outlet of the passage. The residence tube may include insulating means for insulating the contents of the residence tube as it passes through.

The transport fluid nozzle may be annular and circumscribe the passage. The transport fluid nozzle may have an inlet, an outlet and a throat portion intermediate the inlet and the outlet, wherein the throat portion has a cross sectional area which is less than that of the inlet and the outlet.

The apparatus may further comprise a transport fluid supply adapted to supply transport fluid to the transport fluid nozzle.

The apparatus may comprise a plurality of starch activation devices in series and/or parallel with one another, wherein the transport fluid supply is adapted to supply transport fluid to the transport fluid nozzle of each device. The apparatus may comprise a plurality of transport fluid supply lines connecting the transport fluid supply with each nozzle, wherein each transport fluid supply line includes a transport fluid conditioning means. The transport fluid conditioning means may be adapted to vary the supply pressure of the transport fluid to each nozzle.

Alternatively, the apparatus may comprise a dedicated transport fluid supply for each transport fluid nozzle. Each transport fluid supply may include a transport fluid conditioning means. Each conditioning means may be adapted to vary the supply pressure of the transport fluid to each respective nozzle.

The apparatus may further comprise a temperature conditioning unit located between the starch activation device and the second vessel, the temperature conditioning unit adapted to increase the temperature of fluid passing from the device to the second vessel.

The apparatus may further comprise a recirculation pipe adapted to allow fluid recirculation between the outlet of the starch activation device and the first vessel.

The apparatus may further comprise a low shear pump adapted to pump fluid from the hydrating means to the starch activation device.

The heating means may comprise a heated water jacket surrounding the first vessel. Alternatively, the heating means may be remote from the hydrating means.

The insulating means may comprise a heated water jacket surrounding the second vessel. Alternatively, the insulating means may comprise a layer of insulating material covering the exterior of the second vessel.

The apparatus may further comprise first and second agitation means located in the first and second vessels, respectively. The first vessel may include a recirculation means for recirculating slurry from the outlet to an inlet thereof.

A preferred embodiment of the present invention will now be described, by way of example only, with reference to the accompanying drawings, in which:
Figure 1 is a schematic view of a biofuel processing apparatus;
Figure 2 is a longitudinal section view through a starch activation device suitable for use in the apparatus shown in Figure 1; and
Figure 3 shows a graph of the temperature and pressure profile of a slurry as it passes through the device shown in Figure 2.

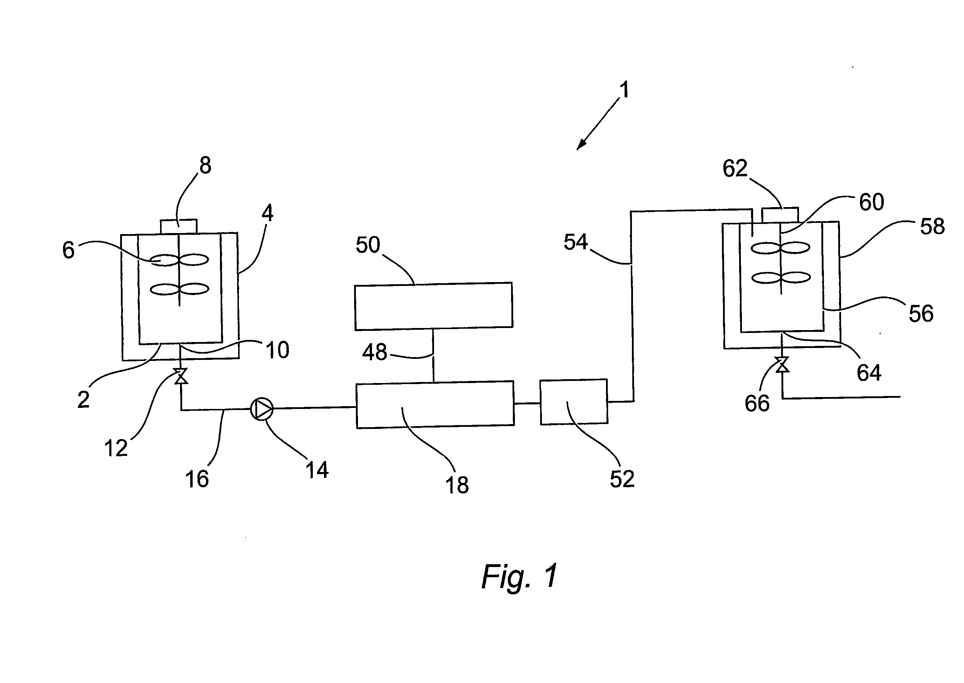

Figure 1 schematically illustrates an apparatus which extracts starch from a starch-based feedstock and then converts the extracted starch into sugars. The apparatus, generally designated 1, comprises a first vessel 2 acting as a first hydrating means. The first vessel 2 has a heating means, which is preferably a heated water jacket 4 which surrounds the vessel 2 and receives heated water from a heated water supply (not shown). The vessel 2 also includes an agitator 6 that is powered by a motor 8. The agitator 6 is suspended from the motor 8 so that it lies inside the vessel 2. At the base of the vessel 2 are an outlet 10 and a valve means 12 which controls fluid flow from the outlet 10. Downstream of the first vessel 2 is a first supply line 16, the upstream end of which fluidly connects to the outlet 10 and valve means 12 whilst the downstream end of the supply line 16 fluidly connects with a reactor 18. A low shear pump 14 may be provided in the supply line 16. The pump 14 may be a centrifugal pump which has been modified in order to reduce shear as fluid is pumped through it.

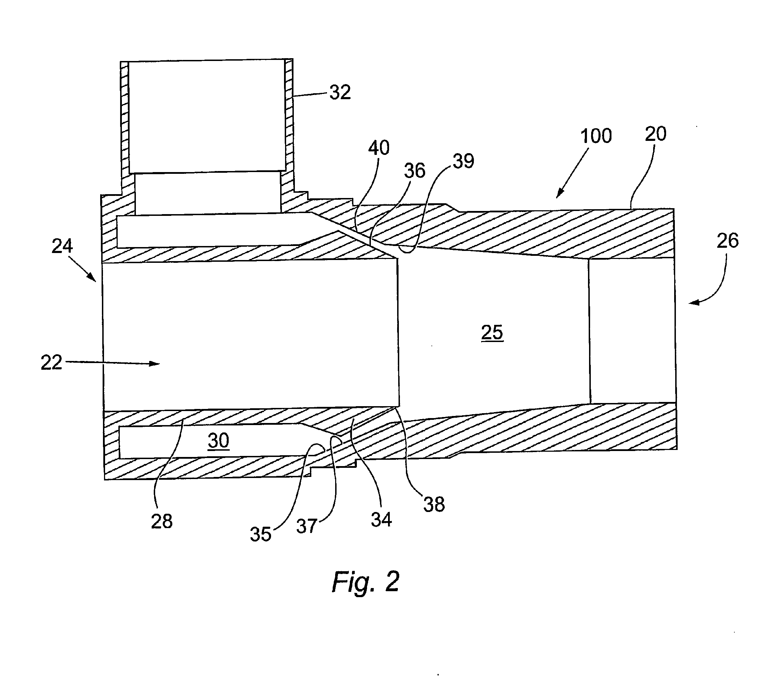

The reactor 18 is formed from one or more starch activation devices. A suitable fluid mover that may act as a starch activation device is shown in detail in Figure 2. The fluid mover 100 comprises a housing 20 that defines a passage 22. The passage 22 has an inlet 24 and an outlet 26, and is of substantially constant diameter. The inlet 24 is formed at the front end of a protrusion 28 extending into the housing 20 and defining exteriorly thereof a plenum 30. The plenum 30 has a transport fluid inlet 32. The protrusion 28 defines internally thereof part of the passage 22. The distal end 34 of the protrusion 28 remote from the inlet 24 is tapered on its relatively outer surface at 36 and defines a transport fluid nozzle 38 between it and a correspondingly tapered part 40 of the inner wall of the housing 20. The nozzle 38 is in fluid communication with the plenum 30 and is preferably annular such that it circumscribes the passage 22. The nozzle 38 has a nozzle inlet 35, a nozzle outlet 39 and a throat portion 37 intermediate the nozzle inlet 35 and nozzle outlet 39. The nozzle 38 has convergent-divergent internal geometry, wherein the throat portion 37 has a cross sectional area which is less than the cross sectional area of either the nozzle inlet 35 or the nozzle outlet 39. The nozzle outlet 39 opens into a mixing chamber 25 defined within the passage 22.

Referring once again to Figure 1, the reactor 18 is connected to a transport fluid supply 50 via a transport fluid supply line 48. The transport fluid inlet 32 of the or each starch activation device 100 making up the reactor is fluidly connected with the transport fluid supply line 48 for the receipt of transport fluid from the transport fluid supply 50.

Located downstream of the reactor 18 and fluidly connected thereto is a temperature conditioning unit (TCU) 52. The TCU 52 preferably comprises a fluid mover substantially identical to that illustrated in Figure 2, and will therefore not be described again in detail here. The TCU 52 can either be connected to the transport fluid supply 50 or else it may have its own dedicated transport fluid supply (not shown).

Downstream of the TCU 52 is a second supply line 54, which fluidly connects the outlet of the TCU 52 with a second vessel 56. The second vessel 56 is similar to the first vessel 2, and therefore has a heated water jacket 58 which surrounds the vessel 56 and receives heated water from a heated water supply (not shown). The vessel 56 also includes an agitator 60 that is powered by a motor 62. The agitator 60 is suspended from the motor 62 so that it lies inside the vessel 56. At the base of the vessel 56 are an outlet 64 and a valve means 66 which controls fluid flow from the outlet 64.

A method of processing starch-based feedstock using the apparatus illustrated in Figures 1 and 2 will now be described in detail. Firstly, a ground starch-based feedstock is introduced into the first vessel 2 at a controlled mass addition flowrate. Examples of suitable feedstock include dry milled maize, wheat or sorghum. Separately, a liquefaction enzyme is mixed with a working fluid, which is water, and that working fluid is then added to the feedstock in the vessel 2 to form a slurry and to start to hydrate the feedstock. An example of a suitable liquefaction enzyme is Liquozyme SC DS® manufactured by Novozymes of Bagsvaerd, Denmark. The preferred enzyme concentration in the vessel 2 is 0.09-0.18 ml/kg. Preferably, the ratio of feedstock to liquid content in the slurry is 20-40% by weight. Optionally, one or more PH adjusters and/or a surfactant can also be added to the slurry at this point.

Heated water is fed into the water jacket 4 surrounding the vessel 2 and the heated water jacket then heats the slurry to a temperature of typically 30-60°C, most preferably 30-40°C, and holds the slurry at this temperature for 30-120 minutes. The motor 8 drives the agitator 6, which stirs the slurry in the vessel 2 with gentle (i.e. low shear) agitation whilst the slurry is held in the vessel 2.

The slurry is held at the desired temperature in the vessel 2 for a sufficient period of time to allow the starch content to be prepared for full hydration. When the slurry has been steeped in the vessel 2 for sufficient time, the valve 12 is opened to allow the slurry to leave the vessel via the outlet 10. The pump 14 pumps the slurry under low shear conditions from the vessel 2 through the first supply line 16 to the reactor 18.

Referring again to Figure 2, when the slurry reaches the or each starch activation device 100 forming the reactor 18, slurry will pass into the device 100 through inlet 24 and out of the outlet 26. Transport fluid, which is steam, is fed from the transport fluid supply 50 at a preferred pressure of between 5-7 Bar to the, or each, transport fluid inlet 32 via transport fluid supply line 48. Introduction of the transport fluid through the inlet 32 and plenum 30 causes a jet of steam to issue forth through the nozzle 38 at a very high, preferably supersonic, velocity. As the steam is injected into the slurry, a momentum and mass transfer occurs between the two which preferably results in the atomisation of the working fluid component of the slurry to form a dispersed droplet flow regime. This transfer is enhanced through turbulence. The steam preferably applies a shearing force to the slurry which not only atomises the working fluid component but also disrupts the cellular structure of the feedstock suspended in the slurry, such that the starch granules present are separated from the feedstock.

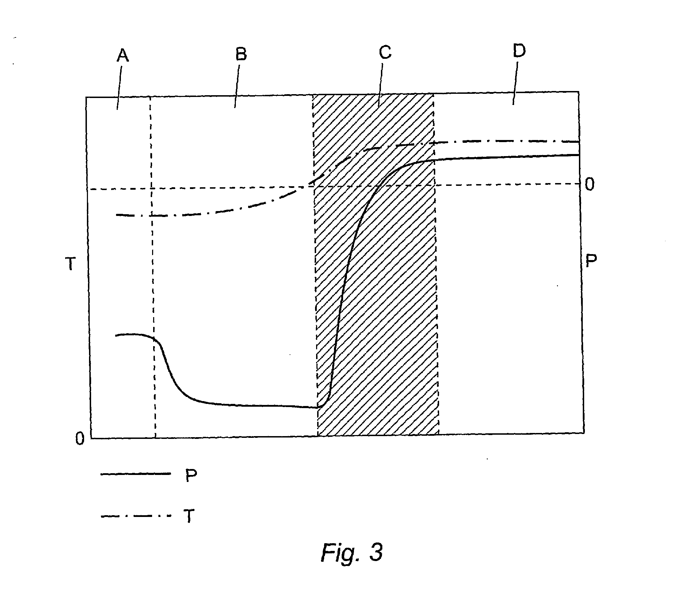

The effects of the process on the temperature and pressure of the slurry can be seen in the graph of Figure 3, which shows the profile of the temperature and pressure as the slurry passes through various points in the fluid mover 100 of Figure 2. The graph has been divided into four sections A-D, which correspond to various sections of the fluid mover 100. Section A corresponds to the section of the passage 22 between the inlet 24 and the nozzle 38. Section B corresponds to the upstream section of the mixing chamber 25 extending between the nozzle 38 and an intermediate portion of the chamber 25. Section C corresponds to a downstream section of the mixing chamber 25 extending between the aforementioned intermediate portion of the chamber 25 and the outlet 26, while section D illustrates the temperature and pressure of the slurry as it passes through the outlet 26.

The steam is injected into the slurry at the beginning of section B of the Figure 3 graph. The speed of the steam, which is preferably injected at a supersonic velocity, and its expansion upon exiting the nozzle 38 may cause an immediate pressure reduction. At a point determined by the steam and geometric conditions, and the rate of heat and mass transfer, the steam may begin to condense, further reducing the pressure and causing an increase in temperature. The steam condensation may continue and form a condensation shock wave in the downstream section of the mixing chamber 25. The forming of a condensation shock wave causes a rapid increase in pressure, as can be seen in section C of Figure 3. Section C also shows that the temperature of the slurry also continues to rise through the condensation of the steam.

As explained above, as the steam is injected into the slurry through nozzle 38 a pressure reduction may occur in the upstream section of the mixing chamber 25. This reduction in pressure forms an at least partial vacuum in this upstream section of the chamber 25 adjacent the nozzle outlet 39. Tests have revealed that an approximately 90% vacuum can be achieved in the chamber 25 as the steam is injected and subsequently condenses.

As previously stated, the shear force applied to the slurry and the subsequent turbulent flow created by the injected steam disrupts the cellular structure of the feedstock suspended in the slurry, releasing the starch granules from the feedstock. As the slurry passes through the partial vacuum and condensation shock wave formed in the chamber 25, it is further disrupted by the changes in pressure occurring, as illustrated by the pressure profile in sections B and C of Figure 3.

As the starch granules are separated from the feedstock in the reactor 18, the granules are almost instantaneously further hydrated, heated and activated due to the introduction of the steam. The device(s) 100 making up the reactor 18 simultaneously pump and heat the slurry and complete the hydration and activate or gelatinise the starch content as the slurry passes through. In addition, the reactor 18 mixes the enzymes with the slurry, providing a homogenous distribution and high level of contact with the starch which is now in a liquid phase. The temperature of the slurry as it leaves the reactor 18 is preferably between 74-76°C. Where the reactor 18 comprises a number of starch activation devices in series, the pressure of the steam supplied to each fluid mover can be individually controlled by a transport fluid conditioning means (not shown) so that the optimum temperature of the slurry is only reached as it exits the last fluid mover in the series. The transport fluid conditioning means may be attached directly to the transport fluid supply 50, or else may be located in the transport fluid supply lines 48.

The temperature at which the slurry leaves the reactor 18 is selected to avoid any heat damage to the slurry contents during the activation stage. However, this temperature may be below the temperature for optimal performance of the liquefaction enzyme, and so the temperature of the slurry may need to be raised without subjecting the slurry to excessively high temperatures or additional shear forces. This gentle heating is achieved using the optional TCU 52 downstream of the reactor 18.

As described above, the TCU 52 comprises one or more fluid movers of the type illustrated in Figure 2. Where there is more than one fluid mover in the TCU 52, they are preferably arranged in series. The pressure of the steam supplied to the fluid mover(s) of the TCU 52 is controlled so that it is comparatively low when compared to that of the steam supplied to the device(s) 100 of the reactor 18. A preferred steam input pressure for the fluid mover(s) of the TCU is between 0.5-2.0 Bar. Consequently, the transport fluid velocity is much lower so no shear force or condensation shock is applied to the slurry by the injected steam as the slurry passes through the TCU 52. Instead, the TCU 52 merely uses the low pressure steam to gently raise the temperature of the slurry.

Once it has passed through the TCU 52, the slurry is preferably at a temperature of between 83-85°C. The slurry then flows downstream through the second supply line 54 into the second vessel 56. The water jacket 58 of the second vessel receives heated water which maintains the slurry at the aforementioned temperature. The slurry is held in the second vessel 56 for a sufficient residence time to allow the enzyme to convert or hydrolyse the starch content into shorter dextrins. During that residence time, the motor 62 drives the agitator 60 to gently agitate the slurry. It has been found that approximately 30 minutes is a sufficient residence time in the present process, compared with a typical residence time of 120 minutes in existing liquefaction processes. The progress of the conversion is monitored during the residence time by measuring the dextrose equivalent (DE) of the slurry. At the end of the residence time, the slurry can be transferred to a fermentation tank (not shown) via the outlet 64 and control valve 66 of the second vessel 56.

Using a starch activation device of the type described allows the present invention to heat and activate the starch content of the slurry while avoiding the creation of regions of extreme heat, which can damage the starch content. Prevention of these regions also reduces or eliminates Maillard effects caused by the reaction of proteins with the extracted starch. These reactions can prevent conversion of the starch to sugar and therefore reduce yields. Furthermore, the gentle agitation mixing and low shear pumping at a lower temperature also ensures that there are no high shear forces which may damage the starch content of the slurry whilst held in a vessel or being transported between vessels. Such damage limits the ultimate glucose yield available from the feedstock.

The starch activation device(s) of the reactor also ensure that the slurry components are more thoroughly mixed than is possible using simple agitator paddles and/or recirculation loops alone. The atomisation of the liquid component of the slurry further ensures a more homogenous mixing of the slurry than previously possible. This improved mixing increases the efficiency of the enzyme in converting starch to shorter dextrins, reducing the time to achieve the desired DE values in the slurry when compared with existing processes.

Alternatively, if desired, this process will achieve higher DE values than possible with existing processes.

The shear action and condensation/pressure shock applied to the feedstock component of the slurry when in the reactor further improves the performance of the present invention as this exposes more of the cellular structure of the feedstock. This allows virtually all the starch granules in the feedstock to be separated, thereby providing improved starch activation rates compared to conventional processes as the enzymatic activation is supplemented by the mechanical activation in the reactor. This also allows the process to provide a starch to sugar conversion ratio of substantially 100%. The process of the present invention therefore may only require the slurry to pass once through the reactor before it is ready to pass to the second vessel for the conversion stage. Hence, yields are much improved as there is no time for loss build up during the process.

Exposing more starch also means that less of the enzyme is needed to achieve the desired DE value of 12-18 before the slurry is transferred to the saccharification and fermentation processes. In addition, the condensation/pressure shock kills bacteria at a relatively low temperature, thereby reducing losses in any subsequent fermentation process.

It has also been discovered that the process and apparatus of the present invention may also improve fermentation rates in the subsequent fermentation process. The improved hydration of the present invention also hydrates some proteins in the feedstock. These hydrated proteins act as additional feedstock to the fermenting yeast, thereby improving the fermenting performance of the yeast.

In summary, the process and apparatus of the present invention have been found to provide a number of advantages over existing arrangements. These advantages include an increase of up to 14% in starch to sugar yields, a reduction of up to 50% of the amount of liquefaction enzyme required, a reduction of up to 75% in the residence time for the conversion to take place, and a reduction of up to 30% in the time taken for the subsequent fermentation of the converted sugars into alcohol.

As described above, the reactor may comprise a plurality of starch activation devices arranged in series and/or parallel. Where the reactor comprises groups of four or more devices in series, the slurry need not be maintained in the desired 30-60°C temperature range whilst being developed in the first vessel. Instead, as each of the devices in the reactor injects high pressure transport fluid into the slurry, the temperature of the slurry as it leaves the first vessel need only be 20-30°C in this instance. An antibiotic additive may be added at the same time as the first enzyme, if desired. Reducing the temperature of the slurry to 20-30°C reduces the requirement for antibiotic infection control.

Whilst the present invention need only utilise one starch activation device in the reactor, if the required process flow rate demands it the reactor may comprise a combination of fluid movers in series and/or parallel. This may also be the case with the temperature conditioning unit made up of one or more of such fluid movers.

The apparatus may also include one or more recirculation pipes which can selectively recirculate slurry from downstream of the starch activation device to upstream of the device, so that the slurry can pass through the device more than once if necessary. Where included, the first vessel may also include such an arrangement so that slurry can pass through the first vessel more than once if necessary.

Instead of having water jackets, the first and/or second vessel may alternatively comprise an insulation layer on the exterior surface thereof. The insulation layer keeps the temperature of the slurry inside the vessel in the desired ranges stated above. The working fluid may be pre-heated by an external heating means (not shown) prior to being mixed with the feedstock. The temperature of the slurry is maintained at the desired temperature in vessel 2 by either using the heated water jacket 4 or the insulation layer.

The low shear centrifugal pump which moves the slurry from the first vessel into the reactor may be replaced with any other suitable low shear pump, such as either a membrane pump or a peristaltic pump, for example.

Whilst the temperature conditioning unit (TCU) described above comprises one or more fluid movers of the type shown in Figure 2, they may be replaced by a heat exchanger. The heat exchanger may be a shell and tube heat exchanger with the slurry passing through a tube and heated water passing through the shell surrounding the tube.

The preferred concentration of the liquefaction enzyme in the slurry during development in the first vessel assumes an average of 15% feedstock moisture content and an average starch content of 75% dry weight.

Whilst the enzyme is preferably introduced to the slurry upstream of the starch activation device, the enzyme may also be introduced in the device or else downstream of the device following activation of the starch content.

Whilst the illustrated embodiment of the invention includes both first and second vessels for handling the slurry, it should be appreciated that the invention need not include the vessels to provide the advantages highlighted above. Instead of a first vessel, the first hydrating means may be a pipe or an in-line mixing device into which the feedstock, working fluid and enzyme are introduced upstream of the starch activation device. Similarly, the second vessel could be replaced by pipework in which the conversion of the activated starch to sugar takes place.

## Claims

1. A process for the treatment of a starch-based feedstock, comprising:
mixing together starch-based feedstock and working fluid to form a slurry;
hydrating the starch-based feedstock with the working fluid;
adding a liquefaction enzyme to the slurry;
pumping the slurry into a substantially constant diameter passage (22) of a starch activation device (100); and
injecting a high velocity transport fluid into the slurry through a nozzle (38) communicating with the passage (22), thereby further hydrating the starch-based feedstock and activating the starch content of the slurry;
wherein the working fluid is water and the transport fluid is steam.

2. The process of Claim 1, wherein the hydrating step includes heating the slurry and/or maintaining it at a first predetermined temperature in the range 30-60°C within a first vessel (2) for a first predetermined period of time.

3. The process of Claim 2 further comprising the step of transferring the slurry to a second vessel (56) from the starch activation device (100), and maintaining the slurry at a second predetermined temperature higher than the first predetermined temperature in the second vessel (56) for a second predetermined period of time.

4. The process of Claim 3, wherein the step of transferring the slurry to the second vessel (56) includes passing the slurry through a temperature conditioning unit (52) to raise the temperature of the slurry from the first predetermined temperature to the second predetermined temperature.

5. The process of Claim 3 or Claim 4 further comprising the step of agitating the slurry in the first and second vessels (2,56) for the respective first and second periods of time.

6. The process of any preceding claim, wherein the steam is injected at a supersonic velocity.

7. The process of any preceding claim, wherein the step of injecting the steam comprises injecting the steam into the slurry through a plurality of nozzles (38) communicating with the passage (22).

8. The process of any preceding claim, wherein the step of injecting the steam into the slurry occurs on a single pass of the slurry through the starch activation device (100).

9. The process of any of Claims 1 to 7, wherein the step of injecting the steam includes recirculating the slurry through the starch activation device (100).

10. The process of any preceding claim, wherein the pumping of the slurry is carried out using a low shear pump (14).

11. An apparatus for treating a starch-based feedstock, the apparatus comprising:
hydrating means for mixing and hydrating the feedstock with a working fluid to form a slurry; and
a starch activation device (100) in fluid communication with the hydrating means;
wherein the starch activation device (100) comprises:
a passage (22) of substantially constant diameter having an inlet (24) in fluid communication with the hydrating means and an outlet (26); and
a transport fluid nozzle (38) communicating with the passage (22) and adapted to inject high velocity transport fluid into the passage (22).

12. The apparatus of Claim 11, wherein the hydrating means comprises a heating means for heating the working fluid and/or the slurry.

13. The apparatus of Claim 12, wherein the hydrating means comprises a first vessel (2) having an outlet (10) in fluid communication with the inlet (24) of the passage (22).

14. The apparatus of Claim 13, wherein the heating means comprises a heated water jacket (4) surrounding the first vessel (2).

15. The apparatus of either Claim 12 or Claim 13, wherein the heating means is remote from the hydrating means.

16. The apparatus of Claim 13 further comprising a second vessel having an inlet in fluid communication with the outlet (26) of the passage (22).

17. The apparatus of Claim 16, wherein the second vessel (56) includes insulating means for insulating the contents of the second vessel (56).

18. The apparatus of any of Claims 11 to 15 further comprising a residence tube section having an inlet in fluid communication with the outlet (26) of the passage (22).

19. The apparatus of Claim 18, wherein the residence tube includes insulating means for insulating the contents of the residence tube as it passes through.

20. The apparatus of any of Claims 11 to 19, wherein the transport fluid nozzle (38) is annular and circumscribes the passage (22).

21. The apparatus of any of Claims 11 to 20, wherein the transport fluid nozzle (38) has an inlet (35), an outlet (39) and a throat portion (37) intermediate the inlet (35) and the outlet (39), wherein the throat portion (37) has a cross sectional area which is less than that of the inlet (35) and the outlet (39).

22. The apparatus of any of Claims 11 to 21 further comprising a transport fluid supply (50) adapted to supply transport fluid to the transport fluid nozzle (38).

23. The apparatus of Claim 22 comprising a plurality of starch activation devices (100) in series and/or parallel with one another, wherein the transport fluid supply (50) is adapted to supply transport fluid to the transport fluid nozzle (38) of each device (100).

24. The apparatus of Claim 23 comprising a plurality of transport fluid supply lines (48) connecting the transport fluid supply (50) with each nozzle (38), wherein each transport fluid supply line (48) includes a transport fluid conditioning means.

25. The apparatus of Claim 24, wherein the transport fluid conditioning means is adapted to vary the supply pressure of the transport fluid to its respective nozzle (38).

26. The apparatus of Claim 22 comprising a dedicated transport fluid supply (50) for each transport fluid nozzle (38).

27. The apparatus of Claim 26, wherein each transport fluid supply (50) includes a transport fluid conditioning means.

28. The apparatus of Claim 27, wherein each conditioning means is adapted to vary the supply pressure of the transport fluid to each respective nozzle (38).

29. The apparatus of any of Claims 11 to 28 further comprising a temperature conditioning unit (52) located downstream of the starch activation device (100), the temperature conditioning unit (52) adapted to increase the temperature of fluid leaving the passage (22) of the device (100).

30. The apparatus of any of Claims 11 to 29 further comprising a recirculation pipe adapted to allow fluid recirculation from downstream of the starch activation device (100) to upstream of the starch activation device (100).

## Patentansprüche

1. Ein Verfahren für die Behandlung eines stärkebasierten Ausgangsmaterials, das Folgendes beinhaltet:
Vermischen von stärkebasiertem Ausgangsmaterial und Arbeitsfluid, um einen Brei zu bilden;
Hydratisieren des stärkebasierten Ausgangsmaterials mit dem Arbeitsfluid; Beigeben eines Verflüssigungsenzyms zu dem Brei;
Pumpen des Breis in einen Durchgang (22) mit im Wesentlichen konstantem Durchmesser einer Stärkeaktivierungsvorrichtung (100); und
Einspritzen eines Hochgeschwindigkeits-Transportfluids durch eine mit dem Durchgang (22) kommunizierende Düse (38) in den Brei und dadurch weiter Hydratisieren des stärkebasierten Ausgangsmaterials und Aktivieren des Stärkegehalts des Breis;
wobei es sich bei dem Arbeitsfluid um Wasser handelt und bei dem Transportfluid um Dampf.

2. Verfahren gemäß Anspruch 1, wobei der Hydratisierungsschritt das Aufheizen des Breis und/oder das Halten desselben auf einer ersten vorherbestimmten Temperatur im Bereich von 30-60 °C in einem ersten Gefäß (2) während eines ersten vorherbestimmten Zeitraums umfasst.

3. Verfahren gemäß Anspruch 2, weiter beinhaltend den Schritt des Überführens des Breis von der Stärkeaktivierungsvorrichtung (100) zu einem zweiten Gefäß (56), und Halten des Breis in dem zweiten Gefäß (56) auf einer zweiten vorherbestimmten Temperatur, die höher ist als die erste vorherbestimmte Temperatur während eines zweiten vorherbestimmten Zeitraums.

4. Verfahren gemäß Anspruch 3, wobei der Schritt des Überführens des Breis zu dem zweiten Gefäß (56) das Leiten des Breis durch eine Temperaturkonditionierungseinheit (52) umfasst, um die Temperatur des Breis von der ersten vorherbestimmten Temperatur auf die zweite vorherbestimmte Temperatur zu erhöhen.

5. Verfahren gemäß Anspruch 3 oder Anspruch 4, weiter beinhaltend den Schritt des Rührens des Breis in dem ersten und in dem zweiten Gefäß (2, 56) während des ersten beziehungsweise des zweiten Zeitraums.

6. Verfahren gemäß einem der vorangehenden Ansprüche, wobei der Dampf mit einer Überschallgeschwindigkeit eingespritzt wird.

7. Verfahren gemäß einem der vorangehenden Ansprüche, wobei der Schritt des Einspritzens des Dampfs das Einspritzen des Dampfs durch eine Vielzahl von mit dem Durchgang (22) kommunizierenden Düsen (38) in den Brei beinhaltet.

8. Verfahren gemäß einem der vorangehenden Ansprüche, wobei der Schritt des Einspritzens des Dampfs in den Brei bei einem einzigen Durchlauf des Breis durch die Stärkeaktivierungsvorrichtung (100) stattfindet.

9. Verfahren gemäß einem der Ansprüche 1 bis 7, wobei der Schritt des Einspritzens des Dampfs das Rückführen des Breis durch die Stärkeaktivierungsvorrichtung (100) umfasst.

10. Verfahren gemäß einem der vorangehenden Ansprüche, wobei das Pumpen des Breis unter Verwendung einer Pumpe (14) mit niedriger Scherung durchgeführt wird.

11. Eine Apparatur zum Behandeln eines stärkebasierten Ausgangsmaterials, wobei die Apparatur Folgendes beinhaltet:
Hydratisierungsmittel zum Mischen und Hydratisieren des Ausgangsmaterials mit einem Arbeitsfluid, um einen Brei zu bilden; und
eine Stärkeaktivierungsvorrichtung (100), die mit dem Hydratisierungsmittel in Fluidverbindung steht;
wobei die Stärkeaktivierungsvorrichtung (100) Folgendes beinhaltet:
einen Durchgang (22) mit im Wesentlichen konstantem Durchmesser, der einen mit dem Hydriermittel in Fluidverbindung stehenden Einlass (24) und einen Auslass (26) aufweist; und
eine Transportfluiddüse (38), die mit dem Durchgang (22) kommuniziert und dazu angepasst ist, Hochgeschwindigkeits-Transportfluid in den Durchgang (22) einzuspritzen.

12. Apparatur gemäß Anspruch 11, wobei das Hydratisierungsmittel ein Aufheizmittel zum Aufheizen des Arbeitsfluids und/oder des Breis beinhaltet.

13. Apparatur gemäß Anspruch 12, wobei das Hydratisierungsmittel ein erstes Gefäß (2) beinhaltet, das einen mit dem Einlass (24) des Durchgangs (22) in Fluidverbindung stehenden Auslass (10) aufweist.

14. Apparatur gemäß Anspruch 13, wobei das Aufheizmittel einen beheizten Wassermantel (4) beinhaltet, der das erste Gefäß (2) umgibt.

15. Apparatur gemäß entweder Anspruch 12 oder Anspruch 13, wobei das Aufheizmittel von dem Hydratisierungsmittel ortsfern ist.

16. Apparatur gemäß Anspruch 13, weiter beinhaltend ein zweites Gefäß, das einen mit dem Auslass (26) des Durchgangs (22) in Fluidverbindung stehenden Einlass aufweist.

17. Apparatur gemäß Anspruch 16, wobei das zweite Gefäß (56) Isoliermittel zum Isolieren des Inhalts des zweiten Gefäßes (56) umfasst.

18. Apparatur gemäß einem der Ansprüche 11 bis 15, weiter beinhaltend einen Verweilröhrenabschnitt, der einen mit dem Auslass (26) des Durchgangs (22) in Fluidverbindung stehenden Einlass aufweist.

19. Apparatur gemäß Anspruch 18, wobei die Verweilröhre Isoliermittel zum Isolieren des Inhalts der Verweilröhre, während er hindurch strömt, umfasst.

20. Apparatur gemäß einem der Ansprüche 11 bis 19, wobei die Transportfluiddüse (38) ringförmig ist und den Durchgang (22) begrenzt.

21. Apparatur gemäß einem der Ansprüche 11 bis 20, wobei die Transportfluiddüse (38) einen Einlass (35), einen Auslass (39) und einen Halsanteil (37) zwischen dem Einlass (35) und dem Auslass (39) aufweist, wobei der Halsanteil (37) eine Querschnittsfläche aufweist, die geringer als diejenige des Einlasses (35) und des Auslasses (39) ist.

22. Apparatur gemäß einem der Ansprüche 11 bis 21, weiter beinhaltend eine Transportfluidzufuhr (50), die dazu angepasst ist, Transportfluid an die Transportfluiddüse (38) zuzuführen.

23. Apparatur gemäß Anspruch 22, beinhaltend eine Vielzahl von in Reihe und/oder parallel zueinander angeordneter Stärkeaktivierungsvorrichtungen (100), wobei die Transportfluidzufuhr (50) dazu angepasst ist, Transportfluid an die Transportfluiddüse (38) jeder Vorrichtung (100) zuzuführen.

24. Apparatur gemäß Anspruch 23, beinhaltend eine Vielzahl von Transportfluid-Zufuhrleitungen (48), die die Transportfluidzufuhr (50) mit jeder Düse (38) verbinden, wobei jede Transportfluid-Zufuhrleitung (48) ein Transportfluid-Konditionierungsmittel umfasst.

25. Apparatur gemäß Anspruch 24, wobei das Transportfluid-Konditionierungsmittel dazu angepasst ist, den Zufuhrdruck des Transportfluids an seine jeweilige Düse (38) zu verändern.

26. Apparatur gemäß Anspruch 22, beinhaltend eine eigene Transportfluidzufuhr (50) für jede Transportfluiddüse (38).

27. Apparatur gemäß Anspruch 26, wobei jede Transportfluidzufuhr (50) ein Transportfluid-Konditionierungsmittel umfasst.

28. Apparatur gemäß Anspruch 27, wobei jedes Konditionierungsmittel dazu angepasst ist, den Zufuhrdruck des Transportfluids an jede jeweilige Düse (38) zu verändern.

29. Apparatur gemäß einem der Ansprüche 11 bis 28, weiter beinhaltend eine stromabwärts von der Stärkeaktivierungsvorrichtung (100) befindliche Temperaturkonditionierungseinheit (52), wobei die Temperaturkonditionierungseinheit (52) dazu angepasst ist, die Temperatur von den Durchgang (22) der Vorrichtung (100) verlassendem Fluid zu erhöhen.

30. Apparatur nach einem der Ansprüche 11 bis 29, weiter beinhaltend ein Rückführungsrohr, das dazu angepasst ist, die Fluidrückführung von stromabwärts von der Stärkeaktivierungsvorrichtung (100) zu stromaufwärts von der Stärkeaktivierungsvorrichtung (100) zuzulassen.

## Revendications

1. Un procédé destiné au traitement d'une charge d'approvisionnement à base d'amidon, comprenant :
mélanger ensemble une charge d'approvisionnement à base d'amidon et un fluide de travail pour former une boue ;
hydrater la charge d'approvisionnement à base d'amidon avec le fluide de travail ; ajouter une enzyme de liquéfaction à la boue ;
pomper la boue dans un passage de diamètre substantiellement constant (22) d'un dispositif d'activation d'amidon (100) ; et
injecter un fluide de transport à grande vitesse dans la boue par une tuyère (38) qui communique avec le passage (22), hydratant dès lors plus avant la charge d'approvisionnement à base d'amidon et activant le contenu amidon de la boue ;
dans lequel le fluide de travail est de l'eau et le fluide de transport est de la vapeur.

2. Le procédé de la revendication 1, dans lequel l'étape d'hydrater consiste à chauffer la boue et / ou la maintenir à une première température prédéterminée comprise dans la gamme allant de 30 à 60 °C dans un premier récipient (2) pendant une première période de temps prédéterminée.

3. Le procédé de la revendication 2 comprenant en outre l'étape de transférer la boue dans un deuxième récipient (56) du dispositif d'activation d'amidon (100), et maintenir la boue à une deuxième température prédéterminée plus élevée que la première température prédéterminée dans le deuxième récipient (56) pendant une deuxième période de temps prédéterminée.

4. Le procédé de la revendication 3, dans lequel l'étape de transférer la boue dans le deuxième récipient (56) consiste à faire passer la boue à travers une unité de conditionnement de température (52) pour élever la température de la boue de la première température prédéterminée à la deuxième température prédéterminée.

5. Le procédé de la revendication 3 ou la revendication 4 comprenant en outre l'étape d'agiter la boue dans les premier et deuxième récipients (2, 56) pendant les première et deuxième périodes de temps respectives.

6. Le procédé de n'importe quelle revendication précédente, dans lequel la vapeur est injectée à une vitesse supersonique.

7. Le procédé de n'importe quelle revendication précédente, dans lequel l'étape d'injecter la vapeur comprend injecter la vapeur dans la boue par une pluralité de tuyères (38) qui communiquent avec le passage (22).

8. Le procédé de n'importe quelle revendication précédente, dans lequel l'étape d'injecter la vapeur dans la boue se produit lors d'un passage unique de la boue à travers le dispositif d'activation d'amidon (100).

9. Le procédé de n'importe lesquelles des revendications 1 à 7, dans lequel l'étape d'injecter la vapeur consiste à faire recirculer la boue à travers le dispositif d'activation d'amidon (100).

10. Le procédé de n'importe quelle revendication précédente, dans lequel le pompage de la boue est effectué en utilisant une pompe à faible cisaillement (14).

11. Un appareil destiné à traiter une charge d'approvisionnement à base d'amidon, l'appareil comprenant :
un moyen d'hydratation destiné à mélanger et hydrater la charge d'approvisionnement avec un fluide de travail pour former une boue ; et
un dispositif d'activation d'amidon (100) en communication fluidique avec le moyen d'hydratation ;
dans lequel le dispositif d'activation d'amidon (100) comprend : un passage (22) de diamètre substantiellement constant ayant une entrée (24) en communication fluidique avec le moyen d'hydratation et une sortie (26) ; et
une tuyère de fluide de transport (38) communiquant avec le passage (22) et adaptée pour injecter du fluide de transport à vitesse élevée dans le passage (22).

12. L'appareil de la revendication 11, dans lequel le moyen d'hydratation comprend un moyen de chauffage destiné à chauffer le fluide de travail et / ou la boue.

13. L'appareil de la revendication 12, dans lequel le moyen d'hydratation comprend un premier récipient (2) ayant une sortie (10) en communication fluidique avec l'entrée (24) du passage (22).

14. L'appareil de la revendication 13, dans lequel le moyen de chauffage comprend une chemise d'eau chauffée (4) qui entoure le premier récipient (2).

15. L'appareil de la revendication 12 ou la revendication 13, dans lequel le moyen de chauffage est éloigné du moyen d'hydratation.

16. L'appareil de la revendication 13 comprenant en outre un deuxième récipient ayant une entrée en communication fluidique avec la sortie (26) du passage (22).

17. L'appareil de la revendication 16, dans lequel le deuxième récipient (56) inclut un moyen isolant destiné à isoler le contenu du deuxième récipient (56).

18. L'appareil de n'importe lesquelles des revendications 11 à 15 comprenant en outre une section de tube de résidence ayant une entrée en communication fluidique avec la sortie (26) du passage (22).

19. L'appareil de la revendication 18, dans lequel le tube de résidence inclut un moyen isolant destiné à isoler le contenu du tube de résidence lorsqu'il le traverse.

20. L'appareil de n'importe lesquelles des revendications 11 à 19, dans lequel la tuyère de fluide de transport (38) est annulaire et circonscrit le passage (22).

21. L'appareil de n'importe lesquelles des revendications 11 à 20, dans lequel la tuyère de fluide de transport (38) a une entrée (35), une sortie (39) et une portion de col (37) entre l'entrée (35) et la sortie (39), dans lequel la portion de col (37) a une surface en coupe transversale qui est inférieure à celle de l'entrée (35) et de la sortie (39).

22. L'appareil de n'importe lesquelles des revendications 11 à 21 comprenant en outre un approvisionnement en fluide de transport (50) adapté pour alimenter la tuyère de fluide de transport (38) en fluide de transport.

23. L'appareil de la revendication 22 comprenant une pluralité de dispositifs d'activation d'amidon (100) en série et / ou en parallèle les uns avec les autres, dans lequel l'approvisionnement en fluide de transport (50) est adapté pour alimenter la tuyère de fluide de transport (38) de chaque dispositif (100) en fluide de transport.

24. L'appareil de la revendication 23 comprenant une pluralité de canalisations d'approvisionnement en fluide de transport (48) raccordant l'approvisionnement en fluide de transport (50) avec chaque tuyère (38), dans lequel chaque canalisation d'approvisionnement en fluide de transport (48) inclut un moyen de conditionnement de fluide de transport.

25. L'appareil de la revendication 24, dans lequel le moyen de conditionnement de fluide de transport est adapté pour faire varier la pression d'approvisionnement du fluide de transport vers sa tuyère respective (38).

26. L'appareil de la revendication 22 comprenant un approvisionnement en fluide de transport dédié (50) pour chaque tuyère de fluide de transport (38).

27. L'appareil de la revendication 26, dans lequel chaque approvisionnement en fluide de transport (50) inclut un moyen de conditionnement de fluide de transport.

28. L'appareil de la revendication 27, dans lequel chaque moyen de conditionnement est adapté pour faire varier la pression d'approvisionnement du fluide de transport vers chaque tuyère respective (38).

29. L'appareil de n'importe lesquelles des revendications 11 à 28 comprenant en outre une unité de conditionnement de température (52) située en aval du dispositif d'activation d'amidon (100), l'unité de conditionnement de température (52) étant adaptée pour augmenter la température du fluide quittant le passage (22) du dispositif (100).

30. L'appareil de n'importe lesquelles des revendications 11 à 29 comprenant en outre un tuyau de recirculation adapté pour permettre une recirculation de fluide de l'aval du dispositif d'activation d'amidon (100) à l'amont du dispositif d'activation d'amidon (100).
